# EUROPÄISCHE PATENTSCHRIFT

(11) **EP 1 310 479 B1**
(45) Veröffentlichungstag und Bekanntmachung des Hinweises auf die Patenterteilung: **27.10.2004**
(21) Anmeldenummer: 03003356.7
(22) Anmeldetag: 26.07.2000
(51) Int. Cl.: C07C 211/51, C07C 209/68, C07C 209/04, C07C 209/30

(54) **Verfahren zur Herstellung von 2-Aminomethyl-1,4-diamino-benzol und dessen Salzen**
Process for the preparation of 2-aminomethyl-1,4-diamino-benzene and salts thereof
Procédé de préparation de 2-aminomethyl-1,4-diamino-benzene et ses sels

(30) Priorität: 18.12.1999 DE 19961229
(43) Veröffentlichungstag der Anmeldung: 14.05.2003
(62) Teilanmeldung aus: 00115959.9
(73) Patentinhaber: Wella Aktiengesellschaft, 64274 Darmstadt (DE)
(72) Erfinder: Göttel, Otto, Dr., 1723 Marly (CH); Pirrello, Aline, 1762 Givisiez (CH); Hayoz, André, 1724 Senèdes (CH)

(56) Entgegenhaltungen:
- WO-A-98/01106
- HANS-JOACHIM KABBE: "Heterocyclen aus Diaminen und Diacylverbindungen" JUSTUS LIEBIGS ANNALEN DER CHEMIE, VERLAG CHEMIE GMBH. WEINHEIM, DE, 1978, Seiten 398-404, XP002184522

## Beschreibung

Gegenstand der vorliegenden Anmeldung ist ein Verfahren zur Herstellung von 2-Aminomethyl-1,4-diamino-2- benzol sowie dessen physiologisch verträglichen Salzen mit organischen oder anorganischen Säuren.

Das 2-Aminomethyl-1,4-diamino-2- benzol wird in Liebigs Annalen der Chemie **1978**, Seite 398-404 als Ausgangsverbindung für die Herstellung von bestimmten Heterozyklen genannt. Ein Verfahren zur Herstellung dieser Verbindung wird jedoch nicht beschrieben. Zudem verliefen Versuche, über das kommerziell erhältliche 2-Amino-5-nitro-benzonitril der Formel (II) durch vollständige Reduktion direkt zur Verbindung der Formel (I) zu gelangen, hinsichtlich Reaktionsbedingungen, Ausbeute und Produktqualität unbefriedigend.

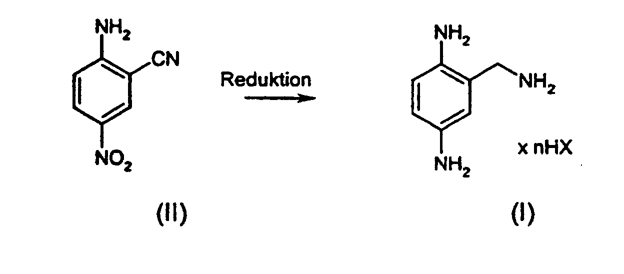

Es bestand daher die Aufgabe, ein einfacheres Herstellungsverfahren für das 2-Aminomethyl-1,4-diamino-2- benzol zur Verfügung zu stellen, das zusätzlich eine einfache Herstellung der oxidationsunempfindlicheren Salze ermöglicht.

Überraschenderweise wurde nunmehr gefunden, dass das 2-Aminomethyl-1,4-diamino-2- benzol durch Aminomethylierung von 4-Nitrophenol, anschließende Umsetzung in das entsprechende Phenoxyacetamid, Umlagerung zum Nitroanilin und anschließende Reduktion auf einfache Weise und in guten Ausbeuten erhältlich ist.

Gegenstand der vorliegenden Verbindung ist daher ein Verfahren zur Herstellung von 2-Aminomethyl-1,4-diamino-benzol oder dessen Salzen der Formel (I), 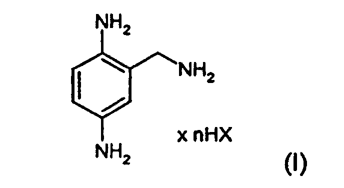 worin n einen Wert von 0 bis 3 aufweist und HX für eine organische oder anorganische Säure steht, in dem zunächst ein 2-(N-Acylaminomethyl)-4-nitrophenol der Formel (III) mit einem Halogenacetamid zum 2-(2-N-Acylamino-methyl-4-nitro-phenoxy)-acetamid der Formel (IV) umgesetzt wird, sodann das 2-(2-N-Acylaminomethyl-4-nitro-phenoxy)-acetamid der Formel (IV) zum 2-(N-Acylaminomethyl)-4-nitroanilin der Formel (V) umgelagert wird, anschließend das 2-(N-Acylaminomethyl)-4-nitroanilin der Formel (V) zum 1,4-Diamino-2-(N-Acylamino-methyl)-benzol der Formel (VI) reduziert wird, sodann mit Salzsäure zum Trihydrochlorid der Formel (VII) deacetyliert wird und gegebenenfalls (wenn n=0) in die freie Base der Formel (I) oder (wenn gilt n≠0 unter der Bedingung, dass gilt HX≠HCl wenn gilt n=3) in ein anderes Salz der Formel (I) überführt wird.

Als Säuren können anorganische oder organische Säuren eingesetzt werden, wobei die folgenden Säuren bevorzugt sind: Salzsäure, Schwefelsäure, Borsäure, Zitronensäure und Weinsäure. Besonders bevorzugt sind Salzsäure und Schwefelsäure.

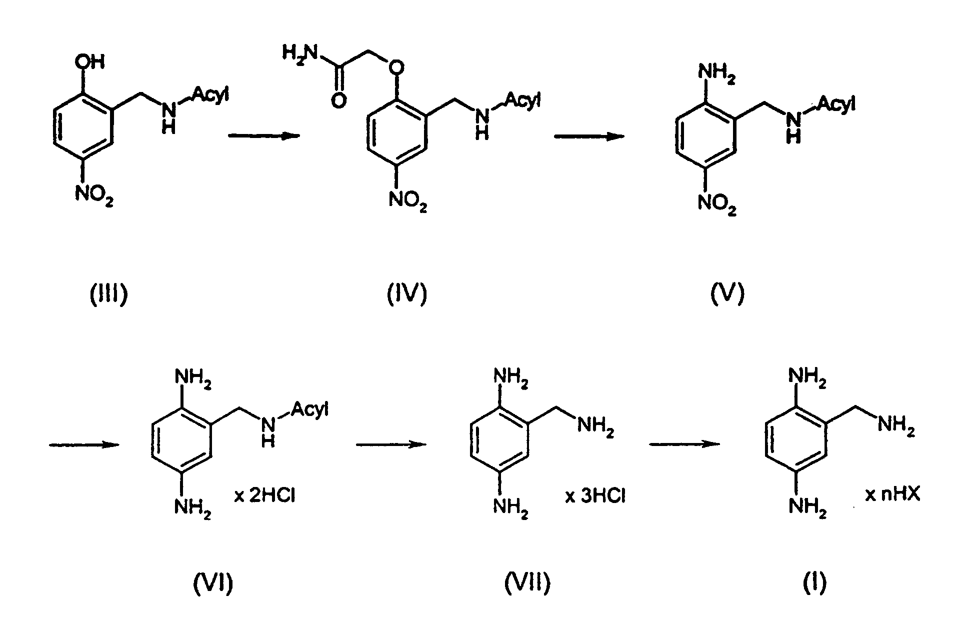

Die Aminomethylierung von 2-(N-Acylaminomethyl)-4-nitrophenol der Formel (III) wurde von H. E. Zaugg, Synthesis **1984**, Seiten 85 - 96, beschrieben. Ausgehend von diesem Zwischenprodukt wird das leicht zugängliche 2-(2-N-Acylamino-methyl-4-nitro-phenoxy)-acetamid der Formel (IV) durch Erwärmen mit einem Halogenoacetamid hergestellt. In idealer Weise eignet sich hierfür Jodacetamid sowie auch das etwas weniger reaktive Bromacetamid. Möchte man aus Kostengründen das billigere Chloracetamid verwenden, so ist es vorteilhaft, die Reaktion mit einem Jodid, beispielsweise Natriumjodid oder Kaliumjodid zu katalysieren. Die zugesetzten Mengen an Jodid können im Hinblick auf eine hohe Umsatzrate weit über 10% (molar) betragen, wobei eine Menge an Jodid von 50% (molar) sich als durchaus angemessen erwiesen hat. Die Reaktion kann in üblicher Weise in einem einfachen dipolaren aprotischen Lösungsmittel wie beispielsweise Aceton unter Rückflusstemperatur durchgeführt werden. Als weitere Lösungsmittel eignen sich Dialkylether des Ethylenglycols und dessen Homologe, wobei Verbindungen mit Siedetemperaturen unter 200°C, insbesondere der Ethylenglycoldimethylether, bevorzugt sind. In Analogie zu W. R. Baker, J. Org. Chem. **1983**, *48*, 5140 erfolgt anschliessend die Umlagerung zum 2-(N-Acylamino-methyl)-4-nitroanilin der Formel (V) in Lösungsmitteln wie Dimethylformamid, Dimethylacetamid oder N-Methyl-pyrrolidon unter Zusatz einer Base. Als Basen eignen sich neben den in der zuvor genannten Literatur genannten Verbindungen auch Carbonate wie Natriumcarbonat oder Kaliumcarbonat. Da die Carbonate unter den Reaktionsbedingungen in Suspension vorliegen, ist es vorteilhaft, sie möglichst feinpulverig und im 2 bis 5fachen Überschuß einzusetzen. Die Reaktion kann in einem breiter Temperaturbereich durchgeführt werden; wobei jedoch zu beachten ist, dass unterhalb von 80°C der Umsatz nur niedrig ist, während oberhalb von 140°C die Reaktionsmischung durch Abbauprodukte dunkel wird. Aus diesem Grunde ist eine Reaktionstemperatur von 80 bis 120°C bevorzugt . Die Verbindung der Formel (V) wird anschliessend nach den üblichen Verfahren, in idealer Weise durch katalytische Hydrierung unter leicht erhöhtem Wasserstoffdruck bei Raumtemperatur oder leicht erhöhter Temperatur, in das 1,4-Diamino-2-(N-acylamino-methyl)-benzol-Dihydrochlorid der Formel (VI) überführt. Zum Schluß erfolgt die Deacylierung in Salzsäure, wobei das 2-Aminomethyl-1,4-diamino-benzol unter den gegebenen Bedingungen als Trihydrochlorid der Formel (VII) anfällt. Dieses Säureaddukt läßt sich in üblicher Weise leicht in die freie Base beziehungsweise andere Säureaddukte der allgemeinen Formel (I) überführen. Selbstverständlich kann auch eine der angeführten Zwischenstufen, beispielsweise Formel (V) dazu verwendet werden.

Infolge der hohen Oxidationsempfindlichkeit der bereits erwähnten freien Base (Formel (I), n=0) wird diese bevorzugt als Säureaddukt (n≠0) der Formel (VII) isoliert, wobei das Trihydrochlorid einen Spezialfall der Formel (I) mit n=3 und HX=HCI darstellt.

Die Verbindungen der Formel (I) eignen sich hervorragend als Farbstoff-Vorstufen im oxidativen System zum Färben von Keratinfasern, wie zum Beispiel Wolle, Seide oder Haaren, insbesondere menschlichen Haaren.

Die nachfolgenden Beispiele sollen den Gegenstand näher erläutern, ohne ihn jedoch auf diese Beispiele zu beschränken.

### Beispiele

### Beispiel 1: Herstellung von 2-Aminomethyl-1,4-diamino-benzoltrihydrochlorid

### Stufe 1: 2-(N-Acetylaminomethyl)-4-nitro-phenoxy-acetamid

131,4 g 2-Acetylaminomethyl-4-nitro-phenol, 64,4 g Chloracetamid, 86,4 g Kaliumcarbonat und 57,1 g Kaliumiodid werden in 800 ml Aceton 4 Stunden lang unter Rückfluß erhitzt. Anschließend läßt man die Reaktionsmischung auf Raumtemperatur abkühlen und gießt auf 1300 ml Wasser. Der ausgefallene Niederschlag wird abfiltriert, mit Wasser gewaschen und anschließend getrocknet. Man erhält 120 g eines gelblichen Produktes mit einem Schmelzpunkt von 236 bis 240°C.

| CHN-Analyse: (C₁₁H₁₃N₃O₅; M = 267,24) | | | |
|---|---|---|---|
| | %C | %H | %N |
| ber. | 49,44 | 4,90 | 15,72 |
| gef. | 49,22 | 4,93 | 15,70 |

### Stufe 2: 2-(N-Acetylaminomethyl)-4-nitroanilin

120 g des 2-(N-Acetylaminomethyl)-4-nitro-phenoxy-acetamids aus Stufe 1 werden in 450 ml N-Methyl-pyrrolidon gelöst, mit 155 g Kaliumcarbonat versetzt und 4,5 Stunden lang auf 100°C erhitzt. Anschließend läßt man die Reaktionsmischung abkühlen, verdünnt mit 840 ml Essigester, filtriert und dampft das Filtrat bei 60-80°C im Vakuum ein. Der Rückstand wird durch Zugabe von Ethanol zur Kristallisation gebracht. Man saugt ab, nimmt die Kristalle in 250 ml Ethanol auf und erhitzt kurz auf Rückfluß. Anschließend kühlt man die Lösung ab, filtriert und trocknet den Rückstand im Vakuum. Es werden 46,6 g eines gelblichen Pulvers mit einem Schmelzpunkt von 208 bis 210°C erhalten.

### Stufe 3: 2-(N-Acetylaminomethyl)-1,4-diamino-benzol-dihydrochlorid

25 g 2-(N-Acetylaminomethyl)-4-nitroanilin aus Stufe 2 werden in 250 ml Ethanol gelöst, mit 2,5 g Palladium (10% auf Aktivkohle) versetzt und unter leicht erhöhtem Wasserstoffdruck hydriert. Nach etwa 5 Stunden ist die Wasserstoffaufnahme beendet und man erhält nach der Entfernung des Katalysators durch Filtration eine farblose Lösung. Durch Zugabe der für die Salzbildung erforderlichen Menge einer gesättigten Lösung von HCI in Ethanol wird das 2-(N-Acetylaminomethyl)-1,4-diamino-benzoldihydrochlorid ausgefällt. Man saugt die Kristalle ab, wäscht mit wenig Ethanol nach und trocknet im Vakuum. Es werden 20,2 g farblose Kristalle mit einem Schmelzpunkt von 215°C (unter Zersetzung) erhalten.

Die Chloridanalyse ergab 27,86% (theoretischer Wert: 28,12%).

### Stufe 4: 2-Aminomethyl-1,4-diamino-benzol-trihydrochlorid

17,7 g des Dihydrochlorids aus Stufe 3 werden in 50 ml Ethanol und 20 ml konzentrierter Salzsäure auf 70°C erhitzt. Man erhält allmählich eine dicke Suspension des Produkts. Nach 3,5 Stunden wird das Reaktionsgemisch auf Raumtemperatur abgekühlt, der Rückstand abgesaugt und gut abgepreßt. Nach dem Trocknen erhält man 12,5 g farblose, hygroskopische Kristalle mit einem Schmelzpunkt von 246 bis 248°C (unter Zersetzung).
¹H-NMR (D₂O): δ= 4,27 ppm (s, 2H); 7,30 ppm (d, ³J_{HH} = 8,55 Hz, 1H); 7,42 ppm (dd, ³J_{HH} = 8,55 Hz, ⁴J_{HH} = 2,60 Hz, 1H); 7,46 ppm (d, ⁴J_{HH} = 2,60 Hz, 1H).
CHNCI-Analyse: (C₇H₁₁N₃ x 2,95 HCI; M = 246,57)
Unter Berücksichtigung eines Restwassergehalts von 6,54% ergibt sich:

| | %C | %H | %N | %CI |
|---|---|---|---|---|
| ber. | 32,11 | 6,10 | 16,05 | 39,94 |
| gef. | 32,3 | 6,00 | 16,1 | 39,9 |

### Beispiel 2: Herstellung von 2-Aminomethyl-1,4-diamino-benzol-disulfat

3 g 2-Aminomethyl-1,4-diamino-benzol-trihydrochlorid aus Beispiel 1 (Stufe 4) werden in 10 ml konzentrierter Schwefelsäure eingerührt, wobei das Salzsäuregas entweicht und eine viskose Masse entsteht. Nach 2 Stunden wird vorsichtig unter schwacher Kühlung mit 50 ml Ethanol verdünnt. Man erhält eine feine Suspension, die 1 Stunde bei Raumtemperatur nachgerührt wird. Absaugen und Nachwaschen mit wenig Ethanol ergibt 2,7 g eines hellgrauen Pulvers mit einem Schemlzpunkt von 242 bis 244°C.
¹H-NMR (DMSO): δ= 3,92 ppm (s, 2H); 6,83 ppm (d, ³J_{HH} = 8,55 Hz, 1H); 7,01 ppm (d, ³J_{HH} = 8,85 Hz); 7,04 ppm (s, verbreitert, 1H).
CHNS-Analyse: (C₇H₁₁N₃ x 2 H₂SO₄; M = 333,35)
Unter Berücksichtigung eines Gehaltes von von 0,5 Mol% Restwasser und 0,1 Mol% Ethanol ergibt sich:

| | %C | %H | %N | %S |
|---|---|---|---|---|
| ber. | 24,92 | 4,82 | 12,11 | 18,48 |
| gef. | 25,40 | 4,70 | 11,90 | 18,60 |

## Patentansprüche

1. Verfahren zur Herstellung von 2-Aminomethyl-1,4-diamino-benzol oder dessen Salzen der Formel (I), 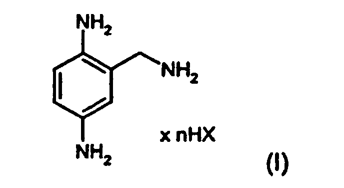 worin n einen Wert von 0 bis 3 aufweist und HX für eine organische oder anorganische Säure steht, in dem zunächst ein 2-(N-Acylaminomethyl)-4-nitrophenol mit einem Halogenacetamid zum 2-(2-N-Acylamino-methyl-4-nitro-phenoxy)-acetamid umgesetzt wird, sodann das 2-(2-N-Acylaminomethyl-4-nitro-phenoxy)-acetamid zum 2-(N-Acylaminomethyl)-4-nitroanilin umgelagert wird, anschließend das 2-(N-Acylaminomethyl)-4-nitroanilin zum 1,4-Diamino-2-(N-Acylamino-methyl)-benzol reduziert wird, sodann mit Salzsäure zum 2-Aminomethyl-1,4-diamino-benzol-Trihydrochlorid deacetyliert wird und das 2-Aminomethyl-1,4-diamino-benzol Trihydrochlorid in die freie Base der Formel (I) (wenn n=0) oder in ein anderes Salz der Formel (I) (wenn n≠0 und HX≠HCl bei n=3) überführt wird.

2. Verfahren nach Anspruch 1, **dadurch gekennzeichnet, dass** die Säure ausgewählt ist aus Salzsäure und Schwefelsäure.

## Claims

1. Process for preparing 2-aminomethyl-1,4-diaminobenzene or its salts of the formula (I) 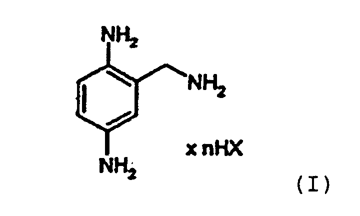 where n has a value of 0 to 3 and HX represents an organic or inorganic acid, in which a 2-(N-acylaminomethyl)-4-nitrophenol is initially reacted with a haloacetamide to give 2-(2-N-acylaminomethyl-4-nitrophenoxy)acetamide, then the 2-(2-N-acylaminomethyl-4-nitrophenoxy)acetamide is rearranged to 2-(N-acylaminomethyl)-4-nitroaniline, then the 2-(N-acylaminomethyl)-4-nitroaniline is reduced to 1,4-diamino-2-(N-acylaminomethyl)benzene, then deacetylated with hydrochloric acid to give 2-aminomethyl-1,4-diaminobenzene trihydrochloride and the 2-aminomethyl-1,4-diaminobenzene trihydrochloride is converted to the free base of the formula (I) (when n = 0) or to another salt of the formula (I) (when n ≠ 0 and HX ≠ HCl when n = 3).

2. Process according to Claim 1, **characterized in that** the acid is selected from hydrochloric acid and sulphuric acid.

## Revendications

1. Procédé pour la préparation de 2-aminométhyl-1,4-diaminobenzène ou ses sels de formule (I) 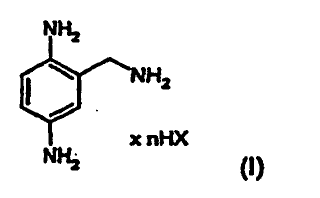 dans laquelle n présente une valeur de 0 à 3 et HX représente un acide organique ou inorganique, dans lequel on transforme d'abord un 2-(N-acylaminométhyl)-4-nitrophénol avec un halogénoacétamide en 2-(2-N-acylaminométhyl-4-nitrophénoxy)acétamide, puis on transpose le 2-(2-N-acylaminométhyl-4-nitrophénoxy)acétamide en 2-(N-acylaminométhyl)-4-nitroaniline, on réduit ensuite la 2-(N-acylaminométhyl)-4-nitroaniline en 1,4-diamino-2-(N-acylaminométhyl)benzène, puis on désacétyle avec de l'acide chlorhydrique en trichlorhydrate de 2-aminométhyl-1,4-diaminobenzène et on transforme le trichlorhydrate de 2-aminométhyl-1,4-diaminobenzène en base libre de formule (I) (lorsque n = 0) ou en un autre sel de formule (I) (lorsque n ≠ 0 et HX ≠ HCl à n = 3).

2. Procédé selon la revendication 1, **caractérisé en ce que** l'acide est choisi parmi l'acide chlorhydrique et l'acide sulfurique.
